# EUROPÄISCHE PATENTSCHRIFT

(11) **EP 0 451 331 B1**
(45) Veröffentlichungstag und Bekanntmachung des Hinweises auf die Patenterteilung: **02.02.1994**
(21) Anmeldenummer: 90121288.6
(22) Anmeldetag: 07.11.1990
(51) Int. Cl.: A45D 34/02, A61L 9/03

(54) **Elektrische Vorrichtung zum Verdampfen von Wirkstoffen**
Electrical device for evaporating substances of efficacy
Dispositif électrique pour l'évaporation de substances d'efficacité

(30) Priorität: 11.04.1990 DE 4011629
(43) Veröffentlichungstag der Anmeldung: 16.10.1991
(73) Patentinhaber: Globol GmbH, D-86633 Neuburg (DE)
(72) Erfinder: Schimaski, Georg, W-5800 Hagen 8 (DE); Hautmann, Horst, W-8858 Neuburg (DE); Fischer, Jürgen, W-8079 Adelschlag (DE)
(74) Vertreter: Skuhra, Udo, Dipl.-Ing.

(56) Entgegenhaltungen:
- EP-A- 0 334 785
- DE-C- 547 130
- FR-A- 1 012 354
- GB-A- 854 266
- GB-A- 1 123 922
- GB-A- 1 123 923

## Beschreibung

Die Erfindung betrifft eine Vorrichtung zum Verdampfen von Wirkstoffen, Parfümen oder dergleichen flüchtigen Substanzen, bestehend aus einem Gehäuse mit einer elektrischen Heizung und einem mit dem Gehäuse verbindbaren Behälter für zu verdampfende Flüssigkeit, wobei im Behälter ein Docht oder dergleichen gehaltert ist, mittels dessen die Flüssigkeit zur Heizung geführt wird, und der Docht die Heizung an einem dem Docht angepaßten Durchgangskanal durchgreift, wobei ferner die Heizung aus einem keramischen Heizkörper mit darin eingelassener elektrischer Heizwendel besteht.

Eine solche Vorrichtung ist bereits aus EP-A-0 334 785 bekannt.

Die bekannten Vorrichtungen sind in vielfacher Hinsicht nachteilig. Insbesondere ist der konstruktive Aufbau sehr aufwendig, da eine Vielzahl von Einzelteilen zusammenzufügen sind, die herstellungstechnisch kompliziert sind. Desweiteren ist nachteilig, daß bei den bekannten gattungsgemäßen Vorrichtungen der Heizkörper als kreisrunde Scheibe ausgebildet ist, die eine Mittellochung aufweist, welche zur Aufnahme des Dochtendes dient. Dabei ist die Heizwendel in diesen Keramikheizkörper dessen Form folgend gebogen eingelegt, was einerseits zu Montageschwierigkeiten führt und andererseits zur Folge hat, daß eine lagerichtige Zuführung und Anordnung der Heizwendel in dem Heizkörper außerordentlich schwierig ist. Zudem ist durch die zwangsläufig vorgesehene Biegung der Heizwendel der Abstand der einzelnen Windungsgänge der Heizwendel relativ innenliegend sehr gering, außenliegend dagegen relativ groß, woraus eine ungleichmäßige Heizbelastung resultiert, die der Lebensdauer der Heizwendel abträglich ist. Desweiteren ist bei den bekannten Vorrichtungen die Dochtausbildung nachteilig, da die ordnungsgemäße Zuführung von Flüssigkeit aus dem Behälter zu der Heizung nicht immer gewährleistet ist, was sich aus der Praxis erwiesen hat.

Ausgehend von diesem Stand der Technik liegt der Erfindung die Aufgabe zugrunde, eine Vorrichtung gattungsgemäßer Art zu schaffen, die bei einfacher Herstellung eine hohe Funktionstüchtigkeit und lange Lebensdauer aufweist.

Zur Lösung dieser Aufgabe wird vorgeschlagen, daß der Heizkörper eine tangential zu seinem Durchgangskanal erstreckende Aufnahme aufweist, die geradlinig ausgebildet ist, in die die Heizwendel eingelegt und die mit Vergußmasse ausgefüllt ist, wobei die elektrischen Anschlußleiter der Heizwendel aus dem Heizkörper etwa koaxial zur Heizwendel herausgeführt sind.

Durch diese Ausbildung ist erreicht, daß der Heizkörper selbst einfacher als bisher herzustellen ist und insbesondere auch eine gleichmäßige Wärmebelastung nach der ordnungsgemäßen Anordnung der Heizwendel erfolgt. Die Anordnung der Heizwendel selbst ist äußerst unproblematisch, da diese lediglich in die geradlinige Aufnahme eingelegt und durch Vergußmasse dort endgültig fixiert werden kann. Es ist selbstverständlich auch möglich, anstelle der Einfachanordnung einer Heizwendel zwei beispielsweise parallel zueinander verlaufende Heizwendeln beidseits des Durchgangskanales in der Fläche des Heizkörpers anzuordnen, wozu dieser dann zwei zueinander parallele Aufnahmen aufweisen müßte. Die Keramische Masse des Heizkörpers selbst bewirkt die Wärmeleitung und somit eine gleichmäßige Wärmeverteilung innerhalb des gesamten Heizkörpers, die durch die Heizwendel hervorgerufen wird, so daß der Durchgangskanal weitestgehend gleichmäßig erwärmt ist und somit eine gleichmäßige Duftstoffabgabe von dem in dem Durchgangskanal befindlichen Dochtende zu erreichen ist.

Eine bevorzugte Weiterbildung wird darin gesehen, daß der Heizkörper eine im wesentlichen dreieckige flache Scheibe ist, nahe deren einer Spitze der Durchgangskanal quer zur Flächenerstreckung der Scheibe verlaufend ausgebildet ist und nahe deren dieser Spitze gegenüberliegender Randkante die Aufnahme für die Heizwendel angeordnet ist.

Diese Ausbildung ist einerseits platzsparend und andererseits auch materialsparend, so daß unter Beibehalt des Montage- und Leistungsvorteils eine kostengünstigere Gestaltung erreicht ist.

Eine bevorzugte Weiterbildung wird darin gesehen, daß die Heizwendel eine Länge aufweist, die etwa dem Durchmesser des Durchgangskanals entspricht.

Besonders bevorzugt ist, daß die Anschlußleiter innerhalb der Aufnahme mit den Anschlußenden der Heizwendel elektrisch leitend verbunden, die Verbindungsstellen ebenso wie die Heizwendel mit Vergußmasse abgedeckt und die isolierten Anschlußleiter aus dem Heizkörper herausgeführt sind.

Hierdurch ist sichergestellt, daß die elektrisch unisolierten Verbindungsstellen durch die Vergußmasse selbst isoliert sind, so daß außerhalb des Heizkörpers lediglich isolierte Anschlußleiter sich befinden.

Um die Vergleichmäßigung der Flüssigkeitsabgabe von dem Behälter bis zur Heizung zu erreichen, wird vorgeschlagen, daß der Docht aus einer Vielzahl von zueinander parallel und in Dochtlängsrichtung verlaufenden, textilen Einzelfäden besteht, die von einer schlauchartigen Gewebehülle zusammengehalten und umgeben sind, wobei die Einzelfäden die Gewebehülle an beiden Dochtenden überragen.

Durch diese Ausbildung ist einerseits eine verbesserte und bleibende Flüssigkeitszufuhr vom Behälter zu der Heizung gewährleistet, wobei andererseits die schlauchartige Gewebehülle sowohl an dem behälterseitigen Ende des Dochtes als auch an dem heizungsseitigen Ende des Dochtes mehr oder weniger abgetrennt werden kann, so daß eine mehr oder weniger große Aufsplittung der einzelnen Fäden des Dochtes an den beiden Enden möglich ist.

Eine weiter bevorzugte Ausbildung wird darin gesehen, daß das Gehäuse aus zwei Halbschalen besteht, deren eine das Gehäuseunterteil mit Behälteranschlußmitteln und deren andere das Gehäuseoberteil bildet, wobei die Heizung zwischen beiden klemmend gehaltert ist.

Diese Anordnung erleichtert die Herstellung die Montage, wobei diese Ausbildung zudem Kostenvorteile hat.

Sofern die Vorrichtung als Steckergerät ausgebildet sein soll, ist es vorteilhaft, wenn die Anschlußleiter zu einem begrenzt drehbar zwischen den Randkanten der Gehäuse-Halbschalen gehalterten Anschlußstecker geführt sind.

Bei einer Ausbildung, bei der die Vorrichtung als Standgerät verwendet wird und demzufolge ein längeres Anschlußkabel zum Anschluß an eine entsprechende Steckdose erforderlich ist, ist es bevorzugt, daß die Anschlußleiter von einer Isolierstoffhülle umgeben, um von vom Gehäuseunterteil zum Oberteil abragende Stifte zick-zack-förmig geführt, und radial aus dem Gehäuse austretend angeordnet sind.

Durch die zick-zack-förmige Führung durch die Gehäusestifte wird eine Zugentlastung des Anschlußkabels (von der Isolierstoffhülle umgebende Anschlußleiter) erreicht, ohne daß hierzu zusätzliche Schraub- oder sonstige Befestigungsmittel notwendig wären

Ferner ist bevorzugt vorgesehen, daß der Behälter an der Dochtaustrittsöffnung mit einem Außengewindehals versehen ist, der in eine entsprechende Gewindeausnehmung des Unterteils einschraubbar ist.

Dabei ist besonders bevorzugt, daß am Hals des Behälters in Einschraubrichtung des Gewindes hintenliegend eine umlaufende Ringrippe und in der Gewindeausnehmung des Gehäuses vornliegend eine Ringnut ausgebildet ist, wobei die Ringrippe bei in Einschraub-Sollage befindlichem Behälter in die Ringnut eingerastet ist.

Durch diese Ausbildung wird eine Rastsicherung erreicht, die sicherstellt, daß der Benutzer merkbar die gewünschte Endposition von Behälter und Gehäuse zueinander einstellt. Beim Einschrauben des Behälters in das Gehäuse erfolgt zunächst eine Drehung innerhalb der Gewindegänge bis die Ringrippe den Bereich erreicht, in welchem die Ringnut angeordnet ist. Die Ringrippe, die vorzugsweise gering über die Gewindeausbildung vorragt greift dann rastend in die entsprechend ausgebildete Ringnut ein, so daß hier durch eine definierte Endlage eingestellt ist, die zusätzlich eine gewisse Kindersicherung bildet.

Es ist selbstverständlich auch möglich, übliche Kindersicherungen bei der Ankopplung von Behälter und Gehäuse vorzusehen.

Eine mögliche Variante wird darin gesehen, daß der Boden des Behälters die Standfläche der Vorrichtung bildet.

Eine unter Umständen bevorzugte Variante besteht darin, daß die Seitenwände des Gehäuseunterteils die Bodenfläche des Behälters überragend ausgebildet sind.

Dabei ist bevorzugt vorgesehen, daß bei in Einschraub-Sollage befindlichem Behälter zwischen der Behälteraußenwandung und der Innenwandung des Gehäuseunterteils ein Luftspalt gebildet ist, der in entsprechende Durchgangsöffnungen des Gehäuses einmündet.

Es ist selbstverständlich ebenso vorteilhaft, wenn die sich auf dem Unterboden abstützenden Seitenwände des Gehäuseunterteiles entweder im Bodenbereich Ausnehmungen zum Eintritt von Luft aufweisen, oder das Gehäuse selbst auch im Bereich des Unterteiles zum Zwecke der Luftzuführung Löcher oder dergleichen besitzt.

Ausführungsbeispiele der Erfindung sind in der Zeichnung dargestellt und im folgenden näher beschrieben.

Es zeigt:
- Fig. 1: Eine erste Ausführungsform der erfindungsgemäßen Vorrichtung in Seitenansicht;
- Fig. 2: desgleichen in um 90° verdrehter Lage;
- Fig. 3: eine Ansicht der Vorrichtung gemäß Fig. 1 von oben gesehen;
- Fig. 4: Einzelteile der Vorrichtung in der Ansicht gemäß Fig. 2;
- Fig. 5: die Einzelteile in der Ansicht gemäß Fig. 1;
- Fig. 6: ein Einzelteil in der Ansicht gemäß Fig. 4 mit eingeschraubtem Behälter;
- Fig. 7: eine andere Ausführungsform in Seitenansicht teilweise geschnitten;
- Fig. 8: die Ausführungsform der Fig. 7 im Schnitt von oben gesehen;
- Fig. 9: eine Einzelheit in Seitenansicht;
- Fig. 10: die Einzelheit in Draufsicht gesehen.

Die elektrische Vorrichtung zum Verdampfen von Wirkstoffen, Parfümen und dergleichen flüchtigen Substanzen besteht im wesentlichen aus einem Gehäuse 1 mit einer elektrischen Heizung 2 und einem mit dem Gehäuse 1 verbindbaren Behälter 3 für zu verdampfende Flüssigkeit. Im Behälter 3 ist ein Docht 4 gehaltert, mittels dessen die Flüssigkeit vom Behälter 3 bis zur Heizung 2 geführt wird. Der Docht 4 durchgreift die Heizung 2 an einem dem Docht angepaßten Durchgangskanal 5. Die Heizung 2 besteht aus einem keramischen Heizkörper 6 mit darin eingelassener elektrischer Heizwendel 7. Wie insbesondere aus Figur 10 ersichtlicht weist der Heizkörper 2 eine tangential zu seinem Durchgangskanal 5 sich erstreckende Aufnahme 8 auf, die geradlinig ausgebildet ist und in welche die Heizwendel 7 ebenfalls geradlinig eingelegt ist. Bei in Sollage befindlichen Einzelteilen wird die Aufnahme 8 mit Vergußmasse ausgefüllt, so daß ein geschlossener Heizkörper 2 entsteht, wie er beispielsweise aus Figur 8 ersichtlich ist. Die elektrischen Anschlußleiter 9 der Heizwendel 7 sind aus dem Heizkörper 2 etwa koaxial zur Heizwendel 7 herausgeführt. In einer bevorzugten Ausführungsform, die in der Zeichnung dargestellt ist, ist der Heizkörper 2 durch eine im wesentlichen dreieckige flache Scheibe gebildet, nahe deren einer Spitze, die im Ausführungsbeispiel abgeflacht ist, der Durchgangskanal quer zur Flächenlängserstreckung der Scheibe verlaufend ausgebildet ist. Nahe der dieser Spitze gegenüberliegenden Randkante der Scheibe ist die Aufnahme 8 für den Heizwendel 7 ausgebildet. Die Heizwendel 7 weist dabei eine solche Länge auf, wie sie etwa dem Durchmesser des Durchgangskanales 5 entspricht. Innerhalb der Aufnahme 8 sind die Anschlußleiter 9 mit den Anschlußenden 10 der Heizwendel 7 elektrisch leitend verbunden, wobei die Verbindungsstellen ebenso wie die Heizwendel 7 mit Vergußmasse abgedeckt wird. Die isolierten Anschlußleiter 9 sind aus dem Heizkörper 2 herausgeführt. Die Ausbildung des Dochtes 4 ist bevorzugt so vorgesehen, daß eine Vielzahl von zueinander parallel und in Dochtlängsrichtung verlaufende textile Einzelfäden von einer schlauchartigen Gewebehülle zusammengehalten und umgeben sind, wobei die Einzelfäden die Gewebehülle an beiden Dochtenden überragen.

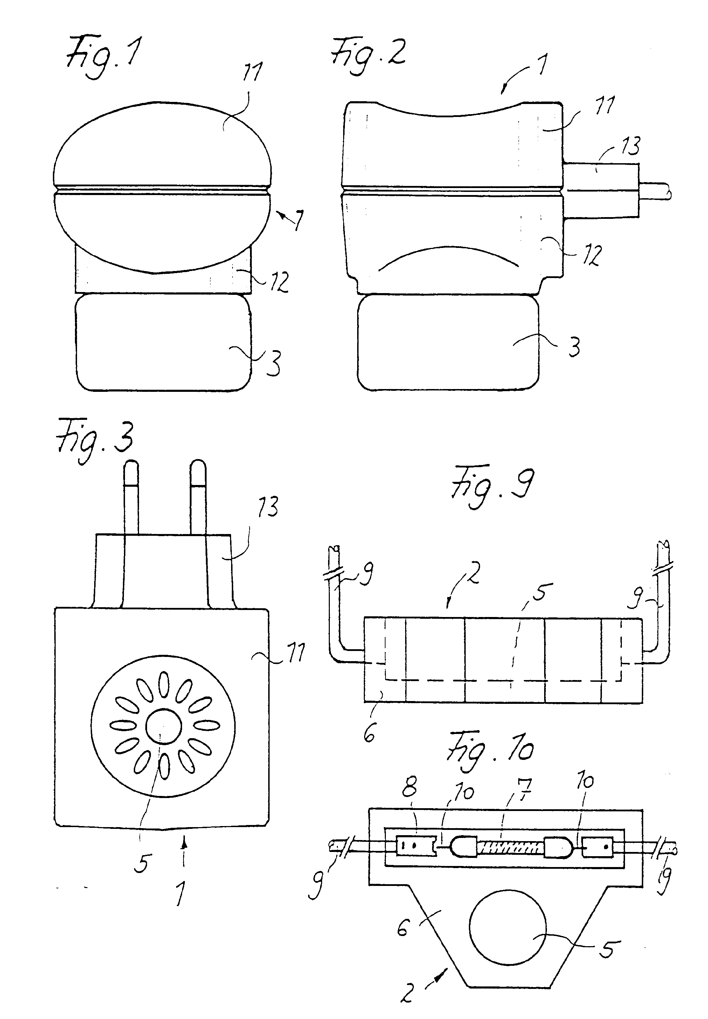

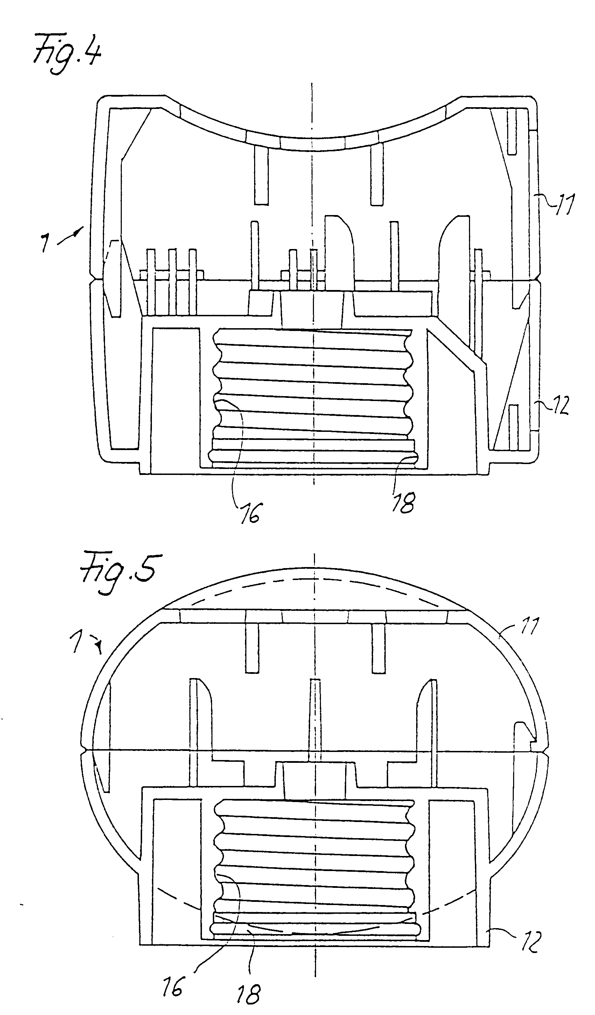

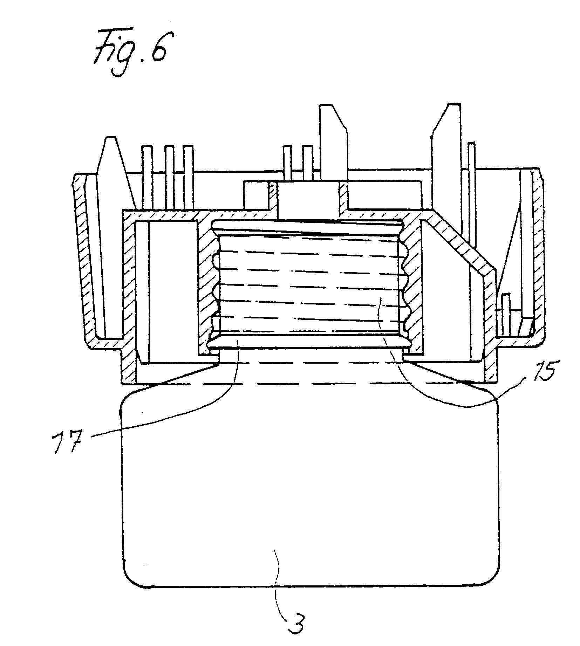

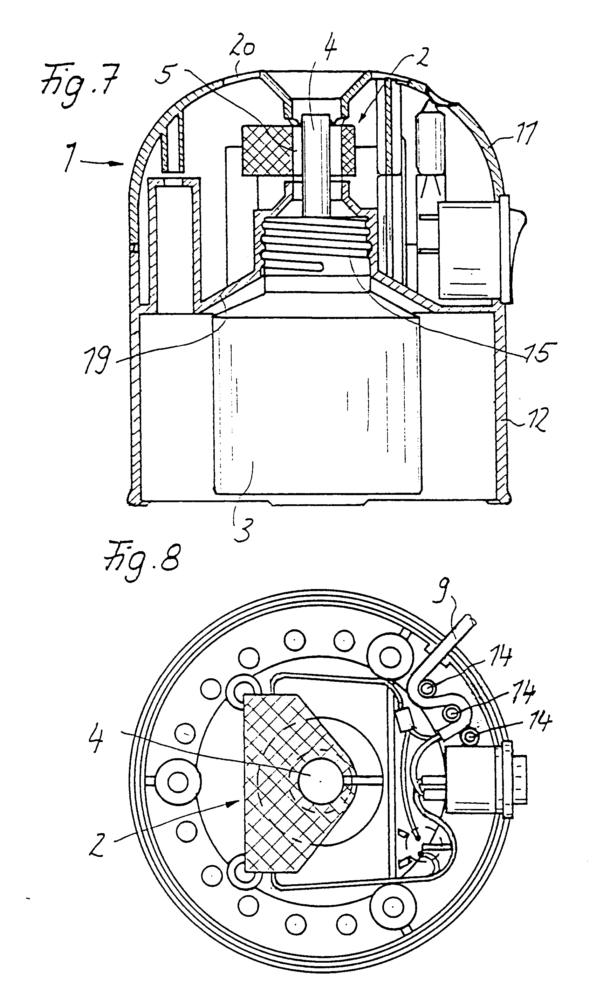

Wie insbesondere aus Figur 4, 5 und 7 ersichtlich, besteht das Gehäuse 1 aus zwei Halbschalen 11, 12, deren eine (12) als Gehäuseunterteil mit Behälteranschlußmitteln und deren andere (11) das Gehäuseoberteil bildet, wobei die Heizung 2 zwischen beiden Gehäuseteilen klemmend eingespannt ist. Die Anschlußleiter 9 sind im Ausführungsbeispiel gemäß Figur 1 bis 3 zu einem begrenzt drehbar zwischen den Randkanten der Gehäusehalbschalen 11, 12 gehalterten Anschlußstecker 13 geführt. Der Anschlußstecker ist so drehbar, daß er einerseits in der Lage gemäß Zeichnung und andererseits in einer um 90° verdrehten Lage auszurichten ist.

Wie insbesondere aus Figur 8 ersichtlich, sind die Anschlußleiter 9 von einer Isolierstoffhülle umgeben und von vom Gehäuseunterteil 12 zum Oberteil 11 hin abragenden Stiften 14 zick-zack-förmig geführt und radial aus dem Gehäuse 1 austretend angeordnet. Durch die zick-zack-förmige Führung über die Stifte 14 wird eine Zugentlastung des Anschlußleiterkabels erreicht.

Wie insbesondere aus den Figuren 6 und 7 ersichtlich, ist der Behälter 3 an der Dochtaustrittsöffnung mit einem Außengewindehals 15 versehen, der in eine entsprechende Gewindeausnehmung 16 des Unterteils 12 einschraubbar ist. Beim Ausführungsbeispiel gemäß Figur 4 bis 6 ist am Hals des Behälters 3 in Einschraubrichtung des Gewindes 15 hintenliegend eine umlaufende Ringrippe 17 vorgesehen, während in der Gewindeausnehmung 16 des Gehäuses vornliegend eine Ringnut 18 ausgeformt ist. Bei in Einschraub-Sollage befindlichen Teilen, wie dies aus Figur 6 ersichtlich ist, ist die Ringrippe 17 in die Ringnut 18 eingerastet. Hierdurch wird ein merkbarer Widerstand gebildet, der einerseits die Einschraubsollposition definiert und andererseits einen Widerstand gegen das Herausschrauben darstellt (Kindersicherung). Wahlweise kann der Boden 3 des Behälters die Standfläche der Gesamtvorrichtung 1 bilden. Beim Ausführungsbeispiel gemäß Figur 7 sind aber die Seitenwände des Gehäuseunterteils 12 die Bodenfläche des Behälters 3 überragend ausgebildet, wobei durch Überstände des Gehäuseseitenwandrandes Luftzuführungsmöglichkeiten gebildet sind. Die Luftzufuhr, die unten am Gehäuseunterteil 12 erfolgt, kann dann über Lochungen oder Spalte 19 und 20 im Gehäuseunterteil bzw. Oberteil erfolgen. Es ist selbstverständlich, daß die Lochungen bzw. Spalte 19, 20 gleichmäßig verteilt angeordnet sind.

## Patentansprüche

1. Elektrische Vorrichtung zum Verdampfen von Wirkstoffen, Parfümen oder dergleichen flüchtigen Substanzen, bestehend aus einem Gehäuse (1) mit einer elektrischen Heizung (2) und einem mit dem Gehäuse (1) verbindbaren Behälter (3) für zu verdampfende Flüssigkeit, wobei im Behälter (3) ein Docht (4) oder dergleichen gehaltert ist, mittels dessen die Flüssigkeit zur Heizung (2) geführt wird, und der Docht (4) die Heizung (2) an einem dem Docht (4) angepaßten Durchgangskanal (5) durchgreift, wobei ferner die Heizung (2) aus einem keramischen Heizkörper (6) mit darin eingelassener elektrischer Heizwendel (7) besteht, **dadurch gekennzeichnet,** daß der Heizkörper (2) eine tangential zu seinem Durchgangskanal (5) erstreckende Aufnahme (8) aufweist, die geradlinig ausgebildet ist, in die die Heizwendel (7) eingelegt und die mit Vergußmasse ausgefüllt ist.

2. Elektrische Vorrichtung nach Anspruch 1, **dadurch** **gekennzeichnet, daß** der Heizkörper (2) eine im wesentlichen dreieckige flache Scheibe ist, nahe deren einer Spitze der Durchgangskanal (5) quer zur Flächenerstreckung der Scheibe verlaufend ausgebildet ist und nahe deren dieser spitze gegenüberliegender Randkante die Aufnahme (8) für die Heizwendel (7) angeordnet ist.

3. Elektrische Vorrichtung nach Anspruch 1 oder 2, **dadurch gekennzeichnet, daß** die Heizwendel (7) eine Länge aufweist, die etwa dem Durchmesser des Durchgangskanals (5) entspricht.

4. Elektrische Vorrichtung nach einem der Ansprüche 1 bis 3, **dadurch gekennzeichnet, daß** die Anschlußleiter (9) innerhalb der Aufnahme (8) mit den Anschlußenden (10) der Heizwendel (7) elektirsch leitend verbunden, die Verbindungsstellen ebenso wie die Heizwendel (7) mit Vergußmasse abgedeckt und die isolierten Anschlußleiter (9) aus dem Heizkörper (2) herausgeführt sind.

5. Elektrische Vorrichtung nach einem der Ansprüche 1 bis 4, **dadurch gekennzeichnet, daß** der Docht (4) aus einer Vielzahl von zueinander parallel und in Dochtlängsrichtung verlaufenden, textilen Einzelfäden besteht, die von einer schlauchartigen Gewebehülle zusammengehalten und umgeben sind, wobei die Einzelfäden die Gewebehülle an beiden Dochtenden überragen.

6. Elektrische Vorrichtung nach einem der Ansprüche 1 bis 5, **dadurch gekennzeichnet, daß** das Gehäuse (1) aus zwei Halbschalen (11, 12) besteht, deren eine das Gehäuseunterteil mit Behälteranschlußmitteln und deren andere das Gehäuseoberteil bildet, wobei die Heizung (2) zwischen beiden klemmend gehaltert ist.

7. Elektrische Vorrichtung nach einem der Ansprüche 1 bis 6, **dadurch gekennzeichnet, daß** die Anschlußleiter (9) zu einem begrenzt drehbar zwischen den Randkanten der Gehäuse-Halbschalen (11, 12) gehalterten Anschlußstecker (13) geführt sind.

8. Elektrische Vorrichtung nach einem der Ansprüche 1 bis 7, **dadurch gekennzeichnet, daß** die Anschlußleiter (9) von einer Isolierstoffhülle umgeben, um vom Gehäuseunterteil (12) zum Oberteil (11) abragende Stifte (14) zick-zack-förmig geführt, und radial aus dem Gehäuse (1) austretend angeordnet sind.

9. Elektrische Vorrichtung nach einem der Ansprüche 1 bis 8, **dadurch gekennzeichnet, daß** der Behälter (3) an der Dochtaustrittsöffnung mit einem Außengewindehals (15) versehen ist, der in eine entsprechende Gewindeausnehmung (16) des Unterteils (12) einschraubbar ist.

10. Elektrische Vorrichtung nach einem der Ansprüche 1 bis 9, **dadurch gekennzeichnet, daß** am Hals des Behälters (3) in Einschraubrichtung des Gewindes (15) hintenliegend eine umlaufende Ringrippe (17) des Gehäuses und vornliegend eine Ringnut (18) ausgebildet ist, wobei die Ringrippe (17) bei in Einschraub-Sollage befindlichem Behälter (3) in die Ringnut (18) eingerastet ist.

11. Elektrische Vorrichtung nach einem der Ansprüche 1 bis 10, **dadurch gekennzeichnet, daß** der Boden des Behälters (3) die Standfläche der Vorrichtung bildet.

12. Elektrische Vorrichtung nach einem der Ansprüche 1 bis 10, **dadurch gekennzeichnet, daß** die Seitenwände des Gehäuseunterteils (12) die Bodenfläche des Behälters (3) überragend ausgebildet sind.

13. Elektrische Vorrichtung nach einem der Ansprüche 1 bis 12, **dadurch gekennzeichnet, daß** bei in Einschraub-Sollage befindlichem Behälter (3) zwischen der Behälteraußenwandung und der Innenwandung des Gehäuseunterteils (12) ein Luftspalt gebildet ist, der in entsprechende Durchgangsöffnungen (19, 20) des Gehäuses (1) einmündet.

14. Elektrische Vorrichtung nach einem der Ansprüche 1 bis 13, **dadurch gekennzeichnet,** daß die elektrischen Anschlußleiter (9) der Heizwendel (7) aus dem Heizkörper (2) etwa koaxial zur Heizwendel (7) herausgeführt sind.

## Claims

1. Electrical apparatus for vaporizing substances of efficacy, perfumes or the like volatile substances, consisting of a housing (1) comprising an electrical heating (2) and a container (3) connectable with the housing (1) for the liquid to be vaporized, whereat in the container (3) a wick (4) or the like is mounted by which the liquid is supplied to the heating (2), and the wick (4) passes through the heating (2) at a through passage (5) adapted to the wick (4), whereat further the heating (2) consists of a ceramic heating body (6) with an electrical heating coil (7) let into said body, **characterized in that** the heating body (2) comprises a recess (8) extending tangentially with respect to its through passage (5), said recess being rectilinear, into which the heating coil (7) is inserted and which is filled with potting composition.

2. Electrical apparatus according to claim 1, characterized in that the heating body (2) is a substantially triangular flat disc, near the one tip of which there is formed a through passage (5) extending transversely to the area extent of the disc and near the edge opposite said tip the recess (8) for the heating coil (7) is arranged.

3. Electrical apparatus according to claim 1 or 2, characterized in that the heating coil (7) has a length which substantially corresponds to the diameter of the through passage (5).

4. Electrical apparatus according to one of the preceding claims 1 to 3, characterized in that the leads (9) within the recess (8) are electrically connected to the terminal ends (10) of the heating coil (7), that the connection points as well as the heating coil (7) are covered with potting composition and the insulated leads (9) are arranged to project from the heating body (2).

5. Electrical apparatus according to one of the preceding claims 1 to 4, characterized in that the wick (4) consists of a plurality of textile filaments extending parallel to each other in longitudinal direction of the wick and are held together and surrounded by a tube-like fabric sheet, whereat the filaments project the fabric sheet beyond the two ends of the wick.

6. Electrical apparatus according to one of the preceding claims 1 to 5, characterized in that the housing (1) consists of two half shells (11, 12) the one of which forms the base comprising container connection means and the other of which forms the housing top, the heating (2) being held clamped between the two shells.

7. Electrical apparatus according to one of the preceding claims 1 to 6, characterized in that the leads (9) are led to the connector plug (13) mounted for limited rotation between the edges of the housing half shells (11, 12).

8. Electrical apparatus according to one of the preceding claims 1 to 7, characterized in that the leads (9) are surrounded by an insulating sheath, led in zig-zag manner around pins (14) projecting from the housing base (12) to the housing top (11) and emerging radially from the housing (1).

9. Electrical apparatus according to one of the claims 1 to 8, characterized in that the container (3) is provided at the wick outlet opening with an outer threaded neck (15) which can be screwed into a corresponding threaded recess (16) of the base portion (12).

10. Electrical apparatus according to one of the claims 1 to 9, characterized in that at the neck of the container (3) lying at the rear in the screw-in direction of the thread (15) an encircling ring rib (17) of the housing is formed and an annular groove (18) is formed at the front, whereat the ring rib (17) engages in the annular groove (18) when the container is in the screwed-in desired position.

11. Electrical apparatus according to one of the claims 1 to 10, characterized in that the bottom of the container (3) forms the standing base of the apparatus.

12. Electrical apparatus according to one of the claims 1 to 10, characterized in that the side walls of the lower part of the housing (12) are formed to project beyond the bottom surface of the container (3).

13. Electrical apparatus according to one of the claims 1 to 12, characterized in that when the container (3) is in the screwed-in desired position between the container outer wall and the inner wall of the lower part of the container (12) an air gap is formed which opens into corresponding through openings (19, 20) of the housing (1).

14. Electrical device according to one of the claims 1 to 13, characterized in that the electrical leads (9) of the heating coil (7) are lead from the heating body (2) substantially coaxially to the heating coil (7).

## Revendications

1. Dispositif électrique de vaporisation de substances actives, parfums ou substances volatiles analogues, constitué d'un boîtier (1), comportant des moyens de chauffage électrique (2), et d'un réservoir (3) agencé de façon à pouvoir être relié au boîtier (1) et destiné à un liquide à vaporiser, tandis qu'il est prévu, maintenue dans le réservoir (3), une mèche (4) ou analogue au moyen de laquelle le liquide est acheminé aux moyens de chauffage (2) et qui traverse ces moyens de chauffage (2) par un conduit de passage (5) adapté à la mèche (4), les moyens de chauffage (2) étant par ailleurs constitués d'un corps de chauffage (6) en céramique dans lequel un enroulement électrique de chauffage (7) est enfermé, caractérisé en ce que le corps de chauffage (2) comporte un logement (8) qui s'étend tangentiellement à son conduit de passage (5) et a une forme rectiligne, dans lequel l'enroulement de chauffage (7) est placé et qui est rempli d'une masse mise en place par coulée.

2. Dispositif électrique suivant la revendication 1, caractérisé en ce que le corps de chauffage (2) est un disque de faible épaisseur et pratiquement triangulaire près d'un sommet duquel le conduit de passage (5) est réalisé en s'étendant perpendiculairement à l'étendue superficielle du disque, tandis que le logement (8) prévu pour l'enroulement de chauffage (7) est disposé près du bord du disque qui est situé à l'opposé dudit sommet.

3. Dispositif électrique suivant l'une des revendications 1 et 2, caractérisé en ce que l'enroulement de chauffage (7) a une longueur qui est approximativement égale au diamètre du conduit de passage (5).

4. Dispositif électrique suivant l'une des revendications 1 à 3, caractérisé en ce que les conducteurs de connexion (9) sont reliés, à l'intérieur du logement (8) et d'une manière électriquement conductrice, aux extrémités de raccordement (10) de l'enroulement de chauffage (7), en ce que les emplacements de raccordement et l'enroulement de chauffage (7) sont recouverts d'une masse mise en place par coulée et en ce que les conducteurs de connexion (9), pourvus d'une isolation, sortent du corps de chauffage (2).

5. Dispositif électrique suivant l'une des revendications 1 à 4, caractérisé en ce que la mèche (4) est constituée d'une multiplicité de fils textiles individuels qui s'étendent parallèlement entre eux et suivant la direction longitudinale de la mèche et qui sont maintenus assemblés et sont entourés par une gaine tissée en forme de tube souple, les fils individuels faisant saillie au-delà de la gaine tissée aux deux extrémités de la mèche.

6. Dispositif électrique suivant l'une des revendications 1 à 5, caractérisé en ce que le boîtier (1) est constitué de deux demi-coquilles (11, 12) dont l'une constitue la partie inférieure de boîtier comportant des moyens de fixation de réservoir et dont l'autre constitue la partie supérieure de boîtier, les moyens de chauffage (2) étant maintenus d'une manière serrée entre les deux parties.

7. Dispositif électrique suivant l'une des revendications 1 à 6, caractérisé en ce que les conducteurs de connexion (9) s'étendent jusqu'à une prise de branchement (13) qui est maintenue, de façon à pouvoir pivoter d'une manière limitée, entre les bords des demi-coquilles (11, 12) du boîtier.

8. Dispositif électrique suivant l'une des revendications 1 à 7, caractérisé en ce que les conducteurs de connexion (9) sont entourés par une gaine de matière isolante, passent en forme de zig-zag autour de tétons (14) faisant saillie sur la partie inférieure (12) du boîtier vers sa partie supérieure (11) et sont disposés de façon à sortir radialement du boîtier (1).

9. Dispositif électrique suivant l'une des revendications 1 à 8, caractérisé en ce qu'à l'endroit de l'ouverture de sortie de la mèche, le réservoir (3) comporte un col extérieurement fileté (15) qui est agencé de façon à pouvoir être vissé dans un évidement fileté correspondant (16) de la partie inférieure (12).

10. Dispositif électrique suivant l'une des revendications 1 à 9, caractérisé en ce qu'une nervure annulaire (17), se refermant sur elle-même, est réalisée sur le col du réservoir (3) en étant disposée vers l'arrière dans le sens du vissage du filetage (15), tandis qu'une gorge annulaire (18) est réalisée dans le logement fileté (16) du boîtier en étant disposée vers l'avant, la nervure annulaire (17) étant emboîtée dans la gorge annulaire (18) lorsque le réservoir (3) est situé dans une position prescrite de vissage.

11. Dispositif électrique suivant l'une des revendications 1 à 10, caractérisé en ce que le fond du réservoir (3) constitue la surface de sustentation du dispositif.

12. Dispositif électrique suivant l'une des revendications 1 à 10, caractérisé en ce que les parois latérales de la partie inférieure (12) du boîtier sont réalisées de façon à faire saillie au-delà de la surface du fond du réservoir (3).

13. Dispositif électrique suivant l'une des revendications 1 à 12, caractérisé en ce que, lorsque le réservoir (3) est situé dans une position prescrite de vissage, il est formé, entre la paroi extérieure du réservoir et la paroi intérieure de la partie inférieure (12) du boîtier, un espace libre qui débouche par des orifices de passage (19, 20) appropriés du boîtier (1).

14. Dispositif électrique suivant l'une des revendications 1 à 13, caractérisé en ce que les conducteurs de connexion (9) de l'enroulement de chauffage (7) sortent du corps de chauffage (2) pratiquement suivant l'axe de l'enroulement de chauffage (7).
